(19) Europäisches Patentamt
European Patent Office
Office européen des brevets

(11) Veröffentlichungsnummer: **0 180 114**
**B1**

(12) # EUROPÄISCHE PATENTSCHRIFT

(45) Veröffentlichungstag der Patentschrift: **08.03.89**

(51) Int. Cl.⁴: **C 07 C 37/62,** C 07 C 39/32

(21) Anmeldenummer: **85113275.3**

(22) Anmeldetag: **19.10.85**

(54) **Verfahren zur Herstellung von Trichlorphenol.**

(30) Priorität: **30.10.84 US 666411**

(43) Veröffentlichungstag der Anmeldung: **07.05.86 Patentblatt 86/19**

(45) Bekanntmachung des Hinweises auf die Patenterteilung: **08.03.89 Patentblatt 89/10**

(84) Benannte Vertragsstaaten: **AT BE CH DE FR GB IT LI NL**

(56) Entgegenhaltungen:
**US-A-3 920 757**
**US-A-4 245 127**
**US-A-4 345 097**

(73) Patentinhaber: **L. GIVAUDAN & CIE Société Anonyme, CH- 1214 Vernier- Genève (CH)**

(72) Erfinder: **Virgilio, Joseph A., 14 Evelyn Terrace, Wayne N.J. 07470 (US)**
Erfinder: **Freudewald, Joachim E., 70 Aspen Drive, Basking Ridge, N.J. 07920 (US)**

(74) Vertreter: **Urech, Peter, Dr., Grenzacherstrasse 124 Postfach 3255, CH- 4002 Basel (CH)**

EP 0 180 114 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die konventionelle industrielle Methode zur Herstellung von 2,4,5-Trichlorphenol (TCP) beinhaltet die Reaktion von 1,2,4,5-Tetrachlorbenzol mit methanolischem oder wässrig-methanolischem Natriumhydroxid bei hohen Temperaturen, d.h. bei ca. 180°C bis 190°C. Der Nachteil dieses Verfahrens besteht darin, dass die Kombination von hoher Temperatur und Alkalinität zur Bildung geringer Mengen von 2,3,7,8-Tetrachlorbenzodioxin (TCDD) führt, welche Verbindung eine der toxischsten Chemikalien darstellt. Auch unter sorgfältigsten Bedingungen kann sich dieses TCDD immer noch in Mengen von 100 ppb oder höher in TCP bilden. Die bei der Herstellung von TCP gebildete Menge von TCDD stellt ein ernstes Umweltproblem dar und hat sogar dazu geführt, dass beispielsweise 2,4,5-T, ein bekanntes Herbizid und Derivat von TCP, nicht mehr verwendet werden kann.

Wenn man deshalb in Zukunft wieder von den vielen Vorteilen Gebrauch machen will, die den Produkten anhaften, die ausgehend aus TCP zugänglich sind, muss demzufolge zuerst ein Verfahren bereitgestellt werden, bei dem TCP in im wesentlichen TCDD-freien Zustand anfällt (unter im wesentlichen TCDD-frei wird eine solche Menge verstanden, die analytisch nicht mehr feststellbar ist, d.h. unter 1 part per billion ist). Die vorliegende Erfindung liefert ein solches Verfahren.

Das Verfahren der vorliegenden Erfindung führt wie gesagt zu TCP, in im wesentlichen TCDD-freien Zustand, unter sauren Bedingungen und bei niedrigen Temperaturen. Das Verfahren beruht auf der überraschenden und unerwarteten Tatsache, dass 2,5-Dichlorphenol mittels Sulfurylchlorid in Anwesenheit von konzentrierter Schwefelsäure und einer katalytischen Menge eines Schwefelkatalysators selektiv chloriert werden kann. Die Reaktion kann wie folgt illustriert werden:

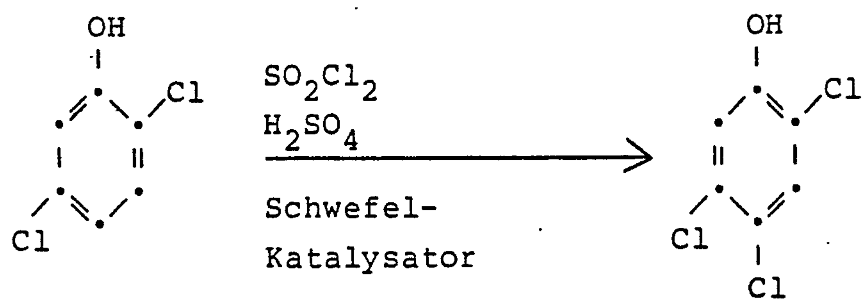


Einfach ausgedrückt, beinhaltet die Reaktion die Monochlorierung von 2,5-Dichlorphenol in 4-Stellung mittels Sulfurylchlorid in Schwefelsäure in Anwesenheit eines Schwefelkatalysators. Ohne diesen Katalysator läuft die Reaktion relativ langsam ab und ist zudem wenig selektiv. Ganz im Gegensatz dazu verläuft die Chlorierung in Anwesenheit des Schwefelkatalysators rasch und hoch selektiv. Wie unten noch erläutert werden wird, stellt der Schwefelkatalysator ein Diarylsulfid dar und die Chlorierung wird bei Temperaturen von bis zu 25°C durchgeführt.

Der Erfolg des vorliegenden Verfahrens beruht darin, dass von einer Reihe von Bedingungen Gebrauch gemacht wird, die zum gewünschten Resultat führen, während dabei gewisse unerwünschte Nebenreaktionen unterdrückt werden. Vorzugsweise wird 1 Mol oder mehr Sulfurylchlorid pro Mol 2,5-Dichlorphenol eingesetzt. Verwendet man weniger als 1 Mol, beispielsweise weniger als 0,9 Mol, tritt im Reaktionsprudukt nicht-umgesetztes 2,5-Dichlorphenol auf. Um sicher zu sein, dass alles Ausgangsmaterial umgesetzt wird, verwendet man vorzugsweise einen geringen Überschuss an Sulfurylchlorid, beispielsweise ungefähr 10 %. Aber auch ein Überschuss von 20 % führt zu sehr guten Resultaten. Ein Überschuss von über 30 % ist nicht ratsam, und zwar darum, weil ein solcher Überschuss zur Chlorierung von 2,4,5-Trichlorphenol zu 2,3,4,6-Tetrachlorphenol, und demzufolge mit niedrigeren Ausbeuten an TCP führt.

Man verwendet vorzugsweise konzentrierte Schwefelsäure. Obschon Konzentrationen so niedrig wie 70 % geeignet sind, erhält man bessere Resultate, wenn die Schwefelsäure konzentrierter ist, also beispielsweise 80 bis 100 %. Die besten Resultate werden mit Schwefelsäurekonzentrationen von 85 - 90 bis 98 % erhalten, und dies ist denn auch der bevorzugte Bereich. Andererseits ist es praktischer und demzufolge noch bevorzugter, die kommerziell erhältliche Schwefelsäure zu verwenden, also eine Schwefelsäure in einer Konzentration von ungefähr 93 bis 96 %.

Es scheint, dass die Menge an eingesetzter Schwefelsäure nicht kritisch ist, obschon es vorteilhaft ist, ein Verhältnis von 1 : 1 (Gewicht/Gewicht) Schwefelsäure zu 2,5-Dichlorphenol zu verwenden, um gute Durchmischung und Fluidität des Gemisches zu gewährleisten: andererseits ist aber auch ein Verhältnis so niedrig wie 0,5 bis 1,0 kommerziell noch möglich. Ein Verhältnis von 1,7 zu 1,0 erscheint optimal. Auch Verhältnisse von bis zu 5,0 : 1,0 liefern gute Resultate, aber die grosse Menge zusätzlich eingesetzter Säure scheint keinerlei Vorteile bezüglich Selektivität zu bringen.

Wie oben gesagt, ist die Anwesenheit eines Schwefelkatalysators äusserst kritisch. Obschon sich eigentlich jede schwefelhaltige organische Verbindung oder auch Schwefel in elementarer Form eignen, scheinen

diejenigen Schwefelverbindungen, bei denen ein aromatischer Ring an das Schwefelatom gebunden ist, am besten zu funktionieren, wobei Diarylsulfide die beste Selektivität und Reaktionsgeschwindigkeit zeitigen.

Jegliches Diarylsulfid erscheint zweckmässig. Besonders bevorzugt ist Diphenylsulfid, da es leicht erhältlich ist und von den bekannteren Diarylsulfiden das ökonomischste ist.

Die Menge der eingesetzten Schwefelverbindung ist nicht kritisch. Man erhält bereits gute Resultate mit geringen Mengen an Katalysator. Es ist aber von Vorteil, ungefähr 0,2 to 0,3 bis 2,0 g Katalysator pro Mol 2,5-Dichlorphenol einzusetzen. Auch grössere Mengen an Katalysator sind möglich, aber eine Menge von über 2,0 g pro Mol 2,5-Dichlorphenol, obschon nicht schädlich, erbringt keine Vorteile mehr.

Ein geeigneter Temperaturbereich ist derjenige zwischen -10°C und +25°C. Unterhalb -40°C beginnt das Gemisch zu gefrieren, und deshalb sind solche tiefen Temperaturen unpraktisch. Bei Temperaturen über +25°C, wird der Wettbewerb zwischen Sulfierung und Chlorierung wesentlich und deshalb fällt das gewünschte Produkt in geringerer Menge an. Es ist vorteilhaft, die Temperatur unterhalb +20°C zu halten, wobei ein Temperaturbereich von zwischen 0°C und 15°C der praktischste und der am meisten bevorzugte ist.

Man kann auch noch zusätzliche Adjuvantien verwenden: So wurde gefunden, dass geringe Mengen an Aluminiumchlorid, beispielsweise 0,1 bis 3,0 g Aluminiumchlorid pro Mol Dichlorphenol, einen positiven, beschleunigenden bzw. kinetischen Effekt auf die Reaktion ausüben. Aber es muss festgehalten werden, dass auch ohne die Verwendung von Aluminiumchlorid gute Selektivität resultiert. Ferner wurde gefunden, dass Dimethylsulfoxid, beispielsweise 20 ml bis 70 ml Dimethylsulfoxid pro Mol Dichlorphenol, einen positiven Effekt auf die Reaktion ausübt, indem diese Menge zusätzliche Fluidität und Durchmischung des Reaktionsgemisches erbringt. Wiederum zeitigt aber die Abwesenheit von Dimethylsulfoxid keinen negativen Einfluss auf die Selektivität der Reaktion.

Die Reaktion kann durchgeführt werden, indem die benötigten Reagentien bei der gewünschten Temperatur gemischt werden. Da die Reaktion exotherm ist, wird die Reaktion vorzugsweise so durchgeführt, dass eines der Reagentien zunächst in das Reaktionsgefäss gegeben wird, das bereits die Schwefelsäure und den Katalysator enthält.

Die Reihenfolge der Zugabe erscheint nicht kritisch. Gute Resultate werden erhalten, wenn entweder Sulfurylchlorid oder 2,5-Dichlorphenol zu den anderen Reagentien gegeben werden, oder wenn beide diese genannten Edukte zur Schwefelsäure und dem Katalysator zugegeben werden. Etwas bessere Resultate werden erhalten, wenn das 2,5-Dichlorphenol zum Reaktionsgemisch gegeben wird, das das Sulfurylchlorid, den Schwefelkatalysator und die Schwefelsäure enthält.

Die Geschwindigkeit der Zugabe ist nicht kritisch und wird aus praktischen Gründen durch die Grösse der Partie und durch die Effizienz der Kühlung bestimmt, beispielsweise sind 30 bis 60 Minuten im Labormaßstab zweckmässig. Hierauf wird das Reaktionsgemisch zweckmässigerweise noch weitergerührt, z. B. während 1 bis 2 Stunden.

Der Ablauf der Reaktion kann verfolgt werden, indem eine kleine Probe aufgearbeitet wird und diese Probe mittels gaschromatographischer Methode analysiert wird, wobei beispielsweise eine Kolonne, wie sie unten in Beispiel 2 beschrieben ist, verwendet wird, und im übrigen ebenfalls von den dort festgehaltenen Bedingungen Gebrauch gemacht wird. Man kann nun die Reaktion beispielsweise auch noch 1 Stunde auf 60° bis 70°C erhitzen, um nicht-umgesetztes 2,5-Dichlorphenol zu sulfieren, dies ist aber nicht unbedingt nötig.

Das Reaktionsgemisch kann aus dem Schwefelsäuregemisch mittels jeder gängiger Methode isoliert werden, beispielsweise, indem man das Schwefelsäuregemisch auf Eis giesst und nun mittels eines geeigneten Lösungsmittels extrahiert, oder indem man ein geeignetes Lösungsmittel zum gekühlten Reaktionsgemisch gibt und hierauf die Lösungsmittelschicht entfernt. Diese letztere Methodik erlaubt, die Schwefelsäure zurückzugewinnen und in einer weiteren Reaktion wiederzuverwenden. Geeignet ist jegliches Lösungsmittel, das im sauren Gemisch unlöslich ist, säurestabil ist und das gebildete 2,4,5-Trichlorphenol solubilisiert: also beispielsweise Kohlenwasserstoffe und zwar aliphatische und aromatische oder chlorierte Kohlenwasserstoffe, beispielsweise Chloroform. Äthylendichlorid und ähnliches. Die Wahl des Lösungsmittels ist nicht kritisch und hängt im wesentlichen davon ab, was mit dem gebildeten TCP geschehen soll.

Beispielsweise wird im Falle wo das 2,4,5-Trichlorphenol als Zwischenprodukt für die Herstellung eines anderen Produktes eingesetzt wird, vorzugsweise in dem Lösungsmittel gearbeitet, das als Lösungsmittel für die nächste Stufe dient, wobei dann diese letztere Lösung tel-quel eingesetzt werden kann. Allerdings ist es ratsam, die angefallene 2,4,5-Trichlorphenollösung vor der weiteren Verwendung neutral zu waschen, insbesondere dann, wenn beispielsweise an die Herstellung von 2,4,5-Trichlorderivaten, wie beispielsweise Hexachlorophen, 2,4,5-T, etc. gedacht wird.

Andererseits kann aber das 2,4,5-Trichlorphenol auch durch einfache Isolation, beispielsweise durch Entfernen des Lösungsmittels, vorzugsweise unter leicht reduziertem Druck, isoliert werden. Es ist angezeigt bei dieser Isolation hohe Temperaturen und Destillationen zu vermeiden, um jegliche Bildung von TCDD zu verhindern.

Es beschreibt zwar auch die US-PS-4 345 097 (Howard) ein Verfahren zur p-Chlorierung von Phenolen, indem substituierte Phenole, z. B. 2,5-Dichlorphenol, mit Sulfurylchlorid in Gegenwart von Schwefel oder Schwefelverbindungen und ggf. noch in Gegenwart eines Friedel-Crafts-Katalysators, z. B. Aluminiumtrichlorid, mit oder ohne Lösungsmittel, selektiv und in hoher Ausbeute p-chloriert werden. Gemäss dieser Literaturstelle beträgt die Menge des anfallenden TCDD's weniger als 100 ppb (Kolonne 5, Zeile 66). Gemäss Beispiel 2 wird, falls das erhaltene 2,4,5-TCP vorerst destilliert wird, das Folgeprodukt 2,4,5-T hierauf weniger als 2 ppb TCDD's aufweisen.

Die Verwendung von Schwefelsäure als Promoter (0,0001 - 1 Gewichtsprozent) für selektive Chlorierungen von Xylenolen geht au US-PS-4 245 127 hervor.

**Beispiel 1**

150 ml 93 %-ige Schwefelsäure und 0,5 g Diphenylsulfid werden in ein 1 l Kolben gegeben und unter Verwendung eines Eisbades auf 5° bis 10°C gekühlt. Man schmelzt 50 g 2,5-Dichlorphenol (55° bis 56°C) und löst in 50 g Sulfurylchlorid. Diese Schmelze wird innert 30 bis 60 Minuten zu einem Reaktionsgemisch gegeben, welches mittels Kühlung durch Eisbad bei 5° bis 10°C gehalten wird. Nach ungefähr 15 bis 30 Minuten fällt 2,4,5-Trichlorphenol als dicke Paste aus. Das Reaktionsgemisch wird 2 weitere Stunden gerührt, hierauf auf 60° bis 70°C eine weitere Stunde erhitzt, um jegliches nicht umgesetzte 2,5-Dichlorphenol zu sulfieren.

Man kühlt hierauf das Reaktionsgemisch auf Zimmertemperatur ab und extrahiert mit 200 ml Äthylendichlorid. Die obere Schicht, die Äthylendichloridschicht, wird abgetrennt und mit 200 ml Wasser und 100 ml 5 %-igem Natriumbicarbonat gewaschen. Das Äthylendichlorid wird auf dem Rotationsverdampfer bei vermindertem Dcuck entfernt, das resultierende Produkt wird gewogen und mittels gaschromatographischer Methodik analysiert, siehe diesbezüglich Beispiel 2, Tabelle 1, Fussnote a.

Gewicht: 58,5 g
Analyse: 2,5-Dichlorphenol in Spuren; 2,3,6-Trichlorphenol = 0,6 %: 2,3,4,6-Tetrachlorphenol = 6,3 %: 2,4,5-Trichlorphenol = 93,1 %.

**Beispiele 2 - 9**

Auf die obige Art und Weise werden die Beispiele 2 - 9 durchgeführt, wobei jedoch jetzt noch zusätzliche Adjuvantien, nämlich Dimethylsulfoxid und Aluminiumchlorid zum Einsatz gelangen, 1,5 g Aluminiumchlorid wird in den 1 l Rundkolben gegeben, zusammen mit der Schwefelsäure und dem Diphenylsulfid. Zu diesem Gemisch gibt man - da die Reaktion exotherm ist - 15 g Dimethylsulfoxid tropfenweise und addiert schliesslich das 2,5-Dichlorphenol und das Sulfurylchlorid. Während dieser ganzen Zeit hält man das Reaktionsgemisch bei +10°C.

In jedem der Beispiele wird eine der Reaktionskomponenten oder eine Reaktionsbedingung variiert, um zu sehen, welchen Effekt dies zeitigt, und gegebenenfalls, welchen Effekt dies auf die Selektivität der Reaktion und den Umsatz von 2,5-Dichlorphenol ausübt. Die anderen Komponenten und/oder Reaktionsbedingungen bleiben wie oben angegeben, d.h. wie in Beispiel 1 festgehalten.

**Beispiel 2**

In diesem Beispiel wird die Menge Diphenylsulfid variiert. Die entsprechenden Resultate sind in der Tabelle I aufgeführt. Es ist sofort ersichtlich, dass ohne die Zugabe des Sulfidkatalysators die Ausbeute extrem niedrig ist, desgleichen die Selektivität.

Schon eine Menge von 0,1 g Diphenylsulfid zeitigt eine dramatische Verbesserung. Die Menge nichtumgesetzten Ausgangsmatetials ist nun sehr klein und die Menge des gewünschten Endproduktes ist höher als 90 %. Durch Zugabe von 0,3 g Diphenylsulfid kann die Selektivität noch erhöht werden, beispielsweise auf eine Ausbeute an 96 % an gewünschtem Produkt. Noch höhere Mengen an Diphenylsulfid scheinen keinen grossen Einfluss mehr zu haben.

**Tabelle I**

| Diphenyl-sulfid | Gewicht Ausbeute | %[a] | | | |
|---|---|---|---|---|---|
| (g) | (g) | A | B | C | D |
| - | 5,1 | 83,4 | 4,8 | 1,2 | 10,6 |
| 0,1 | 58,9 | 0,3 | 2,1 | 5,5 | 92,1 |
| 0,3 | 62,0 | Spur | 1,4 | 2,5 | 96,1 |
| 0,6 | 61,8 | Spur | 1,4 | 2,5 | 96,1 |
| 1,0 | 61,7 | Spur | 0,6 | 2,5 | 96,1 |

a. Die Zusammensetzung wurde mittels gaschromatographischer Methodik bestimmt. Säule 6 Fuss (1 Fuss = 30,48 cm), 3 % FFAP (Free Fatty Acid-Phase), 190° isotherm oder
6 Fuss 5 % SE-30 Kolonne bei 112° bis 210°C bei einer Temperatursteigerung von 10°C/Min.

A: 2,5-Dichlorphenol
B: 2,3,6-Trichlorphenol
C: 2,3,4,6-Tetrachlorphenol
D: 2,4,5-Trichlorphenol

**Beispiel 3**

In diesem Beispiel wird die Konzentration der Schwefelsäure variiert. Zusätzlich wird die Reaktion auch noch in 20 %-igem Oleum durchgeführt. Aus der Tabelle II ist sofort ersichtlich, dass die Menge an Schwefelsäure keinen schwerwiegenden Effekt auf die Selektivität der Reaktion hat, und dass man auch noch mittels 80 %-iger Schwefelsäure gute Selektivität erzielt. Es wird andererseits vermutet. dass Oleum bei 10°C ein zu kräftiges Sulfierungsmittel ist, indem hier keine isolierbaren chlorierten Phenole erhalten werden.

**Tabelle II**

| $H_2SO_4$ Volumen (ml) | Konz. (%) | Gewicht Ausbeute (g) | %[a] A | B | C | D |
|---|---|---|---|---|---|---|
| 150 | 80 | 61,4 | Spur | 10,2 | 2,0 | 87,8 |
| 150 | 85 | 62,0 | Spur | 4,8 | 2,0 | 93,2 |
| 25 | 93 | 60,4 | 1,0 | 6,6 | 2,5 | 89,9 |
| 50 | 93 | 59,9 | Spur | 3,0 | 2,0 | 95,0 |
| 100 | 93 | 61,5 | Spur | 2,0 | 2,0 | 96,0 |
| 150 | 20 % Oleum | - | - | - | - | - |

a. Siehe Tabelle 1, Fussnote a.
b. Kein Reaktionsprodukt.

**Beispiel 4**

In diesem Beispiel wird die Menge Sulfurylchlorid variiert. Es ist aus der Tabelle III sofort ersichtlich, dass die Verwendung von 1 Mol Sulfurylchlorid pro Mol Dichlorphenol das gewünschte Produkt in hoher Ausbeute und guter Selektivität erbringt. Die Verwendung eines geringen Überschusses, d.h. 1,1 Mol pro Mol Dichlorphenol liefert benfalls exzellente Resultate.

Wenn andererseits die Konzentration von Sulfurylchlorid erhöht wird, stellt man Überchlorierung fest, d.h. das 2,4,5-Trichlorphenol wird noch weiter chloriert und man erhält 2,3,4,6-Tetrachlorphenol. Der Anteil an 2,3,4,6-Tetrachlorphenol wird dann unakzeptabel hoch, wenn das Verhältnis von Sulfurylchlorid zu Dichlorphenol wesentlich höher als 1,3 zu 1 ist.

**Tabelle III**

| $SO_2Cl_2$ Gewicht (g) | Molverhältnis[a] | Bewicht Ausbeute (g) | %[b] A | B | C | D |
|---|---|---|---|---|---|---|
| 40 | 1,0 | 60,8 | 1,3 | 3,6 | <1,0 | 95,1 |
| 45 | 1,1 | 61,5 | Spur | 0,6 | 2,5 | 96,0 |
| 50 | 1,2 | 61,0 | Spur | 0,6 | 5,0 | 94,4 |
| 55 | 1,3 | 62,0 | Spur | 2,1 | 8,5 | 89,4 |
| 60 | 1,5 | 62,3 | Spur | 2,2 | 21,6 | 76,1 |

a. Mol $SO_2Cl_2$ pro Mol 2,5-Dichlorphenol.
b. Siehe Tabelle I, Fussnote a.

**Beispiel 5**

In diesem Beispiel wird die Menge Aluminiumchlorid variiert. Aus der untenstehenden Tabelle IV ist ersichtlich, dass die Menge Aluminiumchlorid keinen oder doch nur einen geringen Effekt auf die Selektivität der Reaktion hat.

**Tabelle IV**

| $AlCl_3$ (g) | Gewicht Ausbeute (g) | %[a] A | B | C | D |
|---|---|---|---|---|---|
| - | 61,1 | Spur | 2,7 | 2,5 | 94,8 |
| 0,1 | 61,6 | Spur | 2,3 | 2,5 | 95,2 |
| 0,3 | 61,2 | Spur | 2,3 | 2,5 | 95,2 |
| 0,6 | 61,2 | Spur | 1,1 | 2,5 | 96,4 |
| 1,0 | 61,9 | Spur | 1,2 | 2,5 | 96,3 |

a. Siehe Tabelle I. Fussnote a.

**Beispiel 6**

In diesem Beispiel wird die Menge Dimethylsulfoxid variiert. Aus der entsprechenden Tabelle V ist ersichtlich, dass die Menge Dimethylsulfoxid einen äusserst geringen oder überhaupt keinen Effekt auf die Ausbeute an 2,4,5-Trichlorphenol oder auf die Selektivität der Reaktion hat.

**Tabelle V**

| Dimethyl-sulfoxid (g) | Gewicht Ausbeute (g) | %[a] A | B | C | D |
|---|---|---|---|---|---|
| - | 60,4 | Spur | 0,5 | 6,0 | 93,5 |
| 5 | 61,7 | Spur | 1,0 | 1,5 | 97,4 |
| 10 | 62,0 | Spur | 1,6 | 2,5 | 95,8 |
| 15 | 61,0 | Spur | 0,6 | 2,5 | 96,9 |
| 20 | 61,6 | Spur | 1,4 | 2,5 | 96,1 |
| 25 | 61,6 | 0,6 | 0,9 | 1,5 | 97,0 |
| 40 | 62,2 | Spur | 1,6 | 2,5 | 95,8 |

a. Siehe Tabelle I. Fussnote a.

**Beispiel 7**

In diesem Beispiel werden als Katalysatoren Sulfide ausprobiert, die keine Diarylsulfide darstellen. In diesen Versuchen wird das Reaktionsgemisch 3 Stunden und nicht nur 2 Stunden bei 10°C gehalten, wie oben angegeben. Aus der entsprechenden Tabelle VI ist ersichtlich, dass die meisten Sulfide und sogar elementarer Schwefel einen nützlichen Effekt zeitigen, insofern als die Ausbeute immer besser ist, als wenn übechaupt kein Katalysator verwendet wird. Andererseits kann festgestellt werden, dass Ausbeute und Selektivität schlecht sind, wenn mit den Diarylsulfidversuchen verglichen wird.

**Tabelle VI**

| Sulfid | (g) | Gewicht Ausbeute (g) | %[a] A | B | C | D |
|---|---|---|---|---|---|---|
| $(n\text{-}Bu)_2S$ | (0,5) | 7,2 | 68,5 | 10,2 | 0,7 | 20,6 |
| $(t\text{-}Bu)_2S$ | (0,5) | 8,1 | 62,8 | 8,3 | 0,8 | 28,1 |
| $(CH_3(CH_2)_{11})_2S$ | (0,5) | 10,4 | 74,7 | 6,4 | 1,6 | 17,3 |
| $(Benzyl)_2S$ | (0,5) | 35,3 | 6,7 | 5,2 | 2,7 | 85,4 |
| $Me_2$ | (0,5) | 8,9 | 76,0 | 3,2 | 1,7 | 19,1 |
| S[b] | (1,0) | 33,5 | 2,5 | 1,3 | 5,1 | 91,1 |
| S[b,c] | (1,0) | 35,0 | 74,2 | 1,8 | 0,7 | 23,3 |
| $(PhS)_2$ | (0,5) | 28,1 | - | 8,6 | 1,3 | 90,1 |
| $(PhS)_2$[c] | (0,5) | 61,7 | 57,4 | 0,5 | 0,2 | 41,9 |
| (PhS) FIGFIGFIG[c] | (0,5) | 56,1 | 24,8 | 0,2 | 0,3 | 74,7 |

a. Siehe Tabelle I. Fussnote a.
b. 10°C / 7 Stunden anstelle von 3 Stunden.
c. Nicht auf 70°C aufgeheizt.

**Beispiel 8**

Hier wird nun die Temperatur variiert, und zwar wie folgt: Es wird je ein Versuch bei 0 - 5°C, bei 10 - 15°C und bei 20 - 25°C durchgeführt. Die entsprechenden Resultate sind aus jer Tabelle VII ersichtlich. Es kann aus dieser Tabelle eschlossen werden, dass die höchste Selektivität bei Temperaturen von 0 - 5°C auftritt: ebenfalls noch sehr selektiv erscheinen Reaktionstemperaturen von 10 - 15°C. Wenn andererseits die Temperatur auf 25°C erhöht wird, fällt die Aus-Deute ab und die Reaktion wird weniget selektiv.

Diese niedrigere Ausbeute bei höheren Temperaturen ist wahrscheinlich der Tatsache zuzuschreiben, dass hier Sulfierung mit Chlorierung in Wettbewerb tritt. Die niedrigere Selektivität ist dann wahrscheinlich dem Umstand zuzuschreiben, dass, wenn Sulfierung mit Chlorierung konkurriert, ein Überschuss an Sulfurylchlorid vorhanden ist, welcher mit dem 2,4,5-Trichlorphenol reagiert, wobei nun eine grössere Menge von 2,3,4,6-Tetrachlorphenol gebildet wird.

**Tabelle VII**

| Temperatur (°C) | Gewicht Ausbeute (g) | %[a] A | B | C | D |
|---|---|---|---|---|---|
| 0- 5 | 60,9 | - | 1,2 | 2,5 | 96,3 |
| 10-15 | 61,5 | - | 2,1 | 5,5 | 92,4 |
| 20-25 | 32,5 | 0,8 | 3,2 | 45,2 | 50,8 |

a. Siehe Tabelle I. Fussnote a.

**Beispiel 9**

In diesem Beispiel wird der Modus der Zugabe variiert. Die Mengen an Reagenzien werden verdoppelt - wenn mit dem Oben geschilderten Verfahren verglichen wird (mit der Ausnahme von Sulfurylchlorid, welches in einer Menge von 89 g eingesetzt wird). Die Reaktionstemperatur wird zwischen 5 - 10°C gehalten. Alle Versuche liefern gute Ergebnisse, obschon die Selektivität am besten ist, wenn das Dichlorphenol zum Reaktionsgemisch zugegeben wird, und zwar allein oder zusammen mit dem Sulfurylchlorid.

**Tabelle VIII**

| Modus[a] | Gewicht Ausbeute (g) | %[b] A | B | C | D |
|---|---|---|---|---|---|
| 1 | 121,4 | 0,4 | 0,9 | 3,2 | 95,5 |
| 2 | 122,1 | Spur | 1,4 | 1,5 | 97,1 |
| 3 | 120,8 | Spur | 0,5 | 5,6 | 93,9 |

a. 1. 2,5-Dichlorphenol wird mit dem Sulfurylchlocid gemischt und zum Gemisch der übrigen Komponenten zugegeben [wie im "allgemeinen" Verfahren beschrieben].
2. Man löst das 2,5-Dichlorphenol in Dimethylsulfoxid und gibt das erhaltene Gemisch zu den übrigen Komponenten.
3. Das Sulfurylchlorid wird zum Gemisch der übrigen Komponenten gegeben.
b. Siehe Tabelle I, Fussnote a.

**Patentansprüche**

1. Verfahren zur Herstellung von 2,4,5-Trichlorphenol, dadurch gekennzeichnet, dass man 2,5-Dichlorphenol mit Sulfurylchlorid in Anwesenheit eines Diarylsulfides und Schwefelsäure bei Temperaturen bis zu 25°C zur Umsetzung bringt.

2. Verfahren nach Anspruch 1, dadurch gekennzeichnet. dass man als Diarylsulfid Diphenylsulfid einsetzt.

3. Verfahren nach Anspruch 1 oder 2, dadurch gekennzeichnet, dass man bei Temperaturen zwischen -10°C und +25°C, bzw. bei Temperaturen von 0°C bis +15°C arbeitet.

4. Verfahren nach einem der Ansprüche 1 bis 3, dadurch gekennzeichnet, dass man Schwefelsäure i Konzentrationen von 80 % bis 100 %, vorzugsweise von 85 % bis 98 %, von 90 % bis 98 % oder von 93 % bis 96 % verwendet.

5. Verfahren nach einem der Ansprüche 1 bis 4, dadurch gekennzeichnet, dass man das Sulfurylchlorid in Mengen von 0,9 Mol bis 1,3 Mol, bzw. 1,0 Mol bis 1,3 Mol pro Mol 2,5-Dichlorphenol verwendet.

6. Verfahren nach einem der Ansprüche 1 bis 5, dadurch gekennzeichnet, dass die Menge Diarylsulfid grösser als 0,2 bis 0,3 g pro Mol des eingesetzten 2,5-Dichlorphenols ist.

7. Verfahren nach einem der Ansprüche 1 bis 6, dadurch gekennzeichnet, dass das Verhältnis von Schwefelsäure zu Dichlorphenol ungefähr 0,5 : 1 bis 5 : 1 % (Gewicht/Gewicht) beträgt.

8. Verfahren nach einem der Ansprüche 1 bis 7, dadurch gekennzeichnet, dass zwischen 1,0 und 1,2, bzw. zwischen 1,0 und 1,3 Mole Sulfurylchlorid pro Mol 2,5-Dichlorphenol eingesetzt werden.

9. Verfahren nach einem der Ansprüche 1 bis 8, dadurch gekennzeichnet, dass man als zusätzliches Adjuvans Aluminiumchlorid verwendet.

10. Verfahren nach Anspruch 9, dadurch gekennzeichnet, dass man das Aluminiumchlorid in einer Menge von 0,1 bis 3,0 g pro Mol Dichlorphenol einsetzt.

11. Verfahren nach einem der Ansprüche 1 bis 10, dadurch gekennzeichnet, dass man Dimethylsulfoxid als zusätzliches Adjuvans verwendet.

12. Verfahren nach Anspruch 11, dadurch gekennzeichnet, dass man 20 bis 70 ml Dimethylsulfoxid pro Mol Dichlorphenol verwendet.

13. Verfahren nach einem der Ansprüche 1 bis 12, dadurch gekennzeichnet, dass das Dichlorphenol zu der Schwefelsäure und zum Diarylsulfid zugegeben wird.

14. Verfahren nach einem der Ansprüche 1 bis 13, dadurch gekennzeichnet, dass man das Reaktionsgemisch nach Beendigung der Chlorierung auf ungefähr 60° bis 70°C erhitzt.

**Claims**

1. A process for the manufacture of 2,4,5-trichlorophenol, characterized by reacting 2,5-dichlorophenol with sulphuryl chloride in the presence of a diaryl sulphide and sulphuric acid at temperatures up to 25°C.

2. A process according to claim 1, characterized in that diphenyl sulphide is used as the diaryl sulphide.

3. A process according to claim 1 or 2, characterized in that the reaction is carried out at temperatures between -10°C and +25°C, or at temperatures of 0°C to +15°C.

4. A process according to any one of claims 1 to 3, characterized in that sulphuric acid is used in concentrations of 80 % to 100 %, preferably of 85 % to 98 %, of 90 % to 98 % or of 93 % to 96 %.

5. A process according to any one of claims 1 to 4, characterized in that the sulphuryl chloride is used in amounts of 0.9 mol to 1.3 mol, or 1.0 mol to 1.3 mol per mol of 2,5-dichlorophenol.

6. A process according to any one of claims 1 to 5, characterized in that the amount of diaryl sulphide is

greater than 0.2 to 0.3 g per mol of 2,5-dichlorophenol used.

7. A process according to any one of claims 1 to 6, characterized in that the ratio of sulphuric acid to dichlorophenol is approximately 0.5 : 1 to 5 : 1 % (weight/weight).

8. A process according to any one of claims 1 to 7, characterized in that between 1.0 and 1.2, or between 1.0 and 1.3 mol of sulphuryl chloride are used per mol of 2,5-dichlorophenol.

9. A process according to any one of claims 1 to 8, characterized in that aluminium chloride is used as an additional adjuvant.

10. A process according to claim 9, characterized in that the aluminium chloride is used in an amount of 0.1 to 3.0 g per mol of dichlorophenol.

11. A process according to any one of claims 1 to 10, characterized in that dimethyl sulphoxide is used as an additional adjuvant.

12. A process according to claim 11, characterized in that 20 to 70 ml of dimethyl sulphoxide are used per mol of dichlorophenol.

13. A process according to any one of claims 1 to 12, characterized in that the dichlorophenol is added to the sulphuric acid and to the diaryl sulphide.

14. A process according to any one of claims 1 to 13, characterized in that the reaction mixture is heated to approximately 60° to 70° C after completion of the chlorination.

**Revendications**

1. Procédé de préparation du 2,4,5-trichlorophénol, caractérisé en ce que l'on fait réagir le 2,5-dichlorophénol avec le chlorure de sulfuryle en présence d'un sulfure de diaryle et d'acide sulfurique à des températures allant jusqu'à 25° C.

2. Procédé selon la revendication 1, caractérisé en ce que l'on utilise en tant que sulfure de diaryle le sulfure de diphényle.

3. Procédé selon la revendication 1 ou 2, caractérisé en ce que l'on opère à des températures comprises entre -10 et +25° C, ou à des températures de 0 à +15° C.

4. Procédé selon l'une des revendications 1 à 3, caractérisé en ce que l'on utilise de l'acide sulfurique à des concentrations de 80 à 100 %, de préférence de 85 à 98 %, de 90 à 98 % ou de 93 à 96 %.

5. Procédé selon l'une des revendications 1 à 4, caractérisé en ce que l'on utilise le chlorure de sulfuryle en quantité de 0,9 nole à 1,3 mole ou de 1,0 mole à 1,3 mole par mole de 2,5-dichlorophénol.

6. Procédé selon l'une des revendications 1 à 5, caractérisé en ce que la quantite de sulfure de diaryle est supérieure à une quantité de 0,2 à 0,3 g parmole de 2,5-dichlorophénol mis en oeuvre.

7. Procédé selon l'une des revendications 1 à 6, caractérisé en ce que le rapport acide sulfurique/dichlorophénol est d'environ 0,5 : 1 à 5 : 1 % (poids/poids).

8. Procédé selon l'une des revendications 1 à 7, caractérisé en ce que l'on utilise de 1,0 à 1,2 ou de 1,0 à 1,3 mole de chlorure de sulfuryle par mole de 2,5-dichlorophénol.

9. Procédé selon l'une des revendications 1 à 8, caractérisé en ce que l'on utilise en outre en tant qu'adjuvant du chlorure d'aluminium.

10. Procédé selon la revendication 9, caractérisé en ce que l'on utilise le chlorure d'aluminium en quantité de 0,1 à 3,0 g par mole de dichlorophénol.

11. Procédé selon l'une des revendications 1 à 10, caractérisé en ce que l'on utilise en outre en tant qu'adjuvant du diméthylsulfoxyde.

12. Procédé selon la revendication 11, caractérisé en ce que l'on utilise de 20 à 70 ml de diméthylsulfoxyde par mole de dichlorophénol.

13. Procédé selon l'une des revendications 1 à 12, caractérisé en ce que l'on ajoute le dichlorophénol à l'acide sulfurique et au sulfure de diaryle.

14. Procédé selon l'une des revendications 1 à 13, caractérisé en ce que, lorsque la chloration est terminée, on chauffe le mélange de réaction à une température d'environ 60 à 70° C.